(19) **Europäisches Patentamt / European Patent Office / Office européen des brevets**

(11) **EP 3 667 672 A1**

(12) **EUROPEAN PATENT APPLICATION**
published in accordance with Art. 153(4) EPC

(43) Date of publication:
**17.06.2020 Bulletin 2020/25**

(51) Int Cl.:
**G16B 40/00** (2019.01)

(21) Application number: **18843835.2**

(22) Date of filing: **09.08.2018**

(86) International application number:
**PCT/KR2018/009086**

(87) International publication number:
**WO 2019/031866 (14.02.2019 Gazette 2019/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.08.2017 KR 20170101539**

(71) Applicant: **Ngenebio**
**Seoul 08394 (KR)**

(72) Inventors:
• **JUNG, Kyongyong**
  **Seoul 08210 (KR)**
• **OH, Ensel**
  **Seoul 06561 (KR)**
• **HONG, Chang Bum**
  **Bucheon-si**
  **Gyeonggi-do 14665 (KR)**
• **KIM, Kwang Joong**
  **Sejong 30126 (KR)**

(74) Representative: **Mewburn Ellis LLP**
**Aurora Building**
**Counterslip**
**Bristol BS1 6BX (GB)**

(54) **METHOD FOR DETECTING GENE REARRANGEMENT BY USING NEXT GENERATION SEQUENCING**

(57) The present invention relates to a method for detecting a gene rearrangement on the basis of next generation sequencing (NGS) and, more specifically, to a method for arranging and extracting read data generated by NGS, analyzing the sequence similarity of the extracted read data so as to detect a gene rearrangement present in a cancer sample and, further, detecting the direction of the gene rearrangement, micro-homology sequences and external insertion sequences and positions. According to the method for detecting a gene rearrangement by using NGS, a gene rearrangement can be detected and the direction of the gene rearrangement, micro-homology sequences, external insertion sequences and the position of gene rearrangement can be accurately differentiated into units of base pairs through the reads obtained from the NGS. In addition, a search can be performed even in concordant read pairs, which have not been searched for by a conventional method, such that accuracy is high, and the time required for the detection can be reduced because only regions of genes related to a specific cancer or tumor can be searched for as the priority. Therefore, the method of the present invention is useful for effectively detecting a gene rearrangement in a cancer sample.

Fig. 5

EP 3 667 672 A1

**EP 3 667 672 A1**

**Description**

[Technical Field]

The present invention relates to a method of detecting a gene rearrangement based on next-generation sequencing (NGS), and more specifically to a method including arranging and extracting read data generated by NGS, analyzing the sequence similarity of the extracted read data to detecting a gene rearrangement present in a cancer sample, and further detecting the direction of the gene rearrangement, micro-homology sequences and externally inserted sequences and positions.

[Background Art]

There are a variety of types of mutations occurring in genes. When a part of the base sequence of DNA is changed, deleted or inserted, the number of genes may increase or decrease, and new encounters may occur between genes that do not exist in normal cells. These gene encounters occur when DNA strands of specific sites are broken by external stimuli and then linked again at unexpected locations. When two genes in different chromosomes move to form a fusion gene, the resultant gene is called an "inter-chromosomal fusion gene", and when two genes in the same chromosome move to form a fusion gene, the resultant gene is called an "intra-chromosomal fusion gene" (Rabbitts, T.H. et al. Nature, Vol. 372, pp. 143-1491994).

Most fusion genes have a lethal effect on cell survival and died in a cell stage. However, a gene created by accidental combination may often have abnormal functionality, and thus survive in an inevitable environment satisfying various conditions. When a gene having a strong promoter binds to an upstream of a proto-oncogene, the expression amount thereof greatly increases, or a fusion gene constantly expressed is obtained. Recent research has found that such a fusion gene acts as an oncogene that causes diseases such as blood cancer, lung cancer, colon cancer and schizophrenia (David, R. et al. Cell Physiol. Biochem. Vol. 37(1), pp.77-93, 2015). Such a fusion gene occurs with an incidence of 4% in non-small cell carcinoma (NSCLC), particularly lung adenocarcinoma (LADC), and EML4-ALK (echinoderm microtubule-associated protein-like 4-Anaplastic lymphoma kinase) rearrangement variation, which occurs mainly in young non-smokers, is a fusion gene caused by inversion of chromosome 2. Various EML4-ALK fusion genes exist depending on the length of EML4, and fusion of TRK-fused gene (TFG), kinesin light chain (KLC-1) and kinesin family member 5b (KIF5B) with ALK genes has also been reported. When ALK is activated by higher genes, it acts as a cause of cancer by facilitating cell proliferation and suppressing apoptosis through signaling systems such as RAS, PI3K and JAK-STAT3 (Takashi, K. et al. Ann Oncol. Vol. 25(1), pp. 138-42. 2014) .

It is considerably important to identify genome rearrangements on somatic cells through NGS data in order to find the cause of cancer resulting from the fusion gene. Paired-end sequencing is commonly used to identify genome rearrangements or structural variants (SVs). There are several methods to detect genome rearrangements from such sequencing data. Among them, a read depth (RD) method is mainly capable of acquiring variation information of the number of gene copies. The resolution is determined by the depth of coverage and the window size. The method is available for both single-end reads and paired-end reads. However, this method has a disadvantage in that the fusion break point where the fusion gene is actually created is not identified in detail (Feuk, L. et al. Nat Rev Genet. Vol. 7(2), pp. 85-97. 2006) .

An abnormal paired-end arrangement method is a method of finding the fusion break point through discordant read pairs in which mate-reads are mapped to different genes and different chromosomes. This method is used when the break point of the fusion gene is present in an unsequenced region between read pairs, and in accordance with this method, resolution is determined by the fragment size and coverage of the read (Chiang, D.Y. et al. Nat Methods. Vol.6(1), pp. 99-103 2009).

The split-read (SR) method is a method of finding a break point by soft-clipping a portion of the read in an alignment program when mapping the read to a reference genome in the case where there is a fusion break point inside the read. This method can be used for both single-end reads and paired-end reads, and is capable of acquiring more accurate results when used for paired-end reads. However, the method has a disadvantage in that a soft-clipped sequence portion may be formed, either by a sequencing error or by micro-homology (Chen, K. et al. Nat Methods.Vol.6(9) pp.677-81. 2009).

Accordingly, as a result of extensive effort to solve the problems with the methods, the present inventors found that, when performing a pair-blast analysis including classifying reads into discordant read pairs and concordant read pairs and then performing comparative analysis using each read as a query, it is possible to detect not only a gene rearrangement, which cannot be detected by other methods, but also the location of the gene rearrangement, the microhomology sequence, and the directions of the external insertion sequence and the gene rearrangement. Based on this finding, the present invention has been completed.

[Disclosure]

[Technical Problem]

It is one object of the present invention to provide a method of detecting a gene rearrangement using next-generation sequencing (NGS).

It is another object of the present invention to provide a computer system including a computer-readable medium encoded with a plurality of instructions for controlling a computing system to perform a method of detecting a gene rearrangement using next-generation sequencing (NGS).

[Technical Solution]

In accordance with one aspect of the present invention, the above and other objects can be accomplished by the provision of a method of detecting a gene rearrangement in a sample including: (a) acquiring a library including a plurality of nucleic acid molecules from a sample of a subject; (b) bringing the library into contact with a plurality of bait sets to enrich the library with respect to a preselected sequence to provide a selected nucleic acid molecule and thereby provide a library catch; (c) acquiring reads from the nucleic acid molecules of the library catch through next-generation sequencing; (d) arranging the reads by an alignment method; and (e) analyzing the arranged reads to detect a gene rearrangement, wherein the analysis method includes extracting the arranged reads to analyze sequence similarity.

In accordance with another aspect of the present invention, provided is a computer system including a computer-readable medium encoded with a plurality of instructions for controlling a computing system to perform a method of detecting a gene rearrangement using next-generation sequencing (NGS),
wherein the method includes: (a) acquiring a library including a plurality of nucleic acid molecules from a sample of a subject; (b) bringing the library into contact with a plurality of bait sets to enrich the library with respect to a preselected sequence to provide a selected nucleic acid molecule and thereby provide a library catch; (c) acquiring reads from the nucleic acid molecules of the library catch through next-generation sequencing; (d) arranging the reads by an alignment method; and (e) analyzing the arranged reads to detecting a gene rearrangement, wherein the analysis method includes extracting the arranged reads to analyze sequence similarity.

[Description of Drawings]

FIG. 1 is a schematic diagram illustrating a gene rearrangement detection mechanism of the present invention.

FIG. 2 is a schematic diagram illustrating the type of read extracted in an embodiment of the present invention.

FIG. 3 is a schematic diagram illustrating a method of analyzing concordant pairs and discordant pairs in an embodiment of the present invention.

FIG. 4 is a schematic diagram illustrating a method of detecting a gene rearrangement in an embodiment of the present invention.

FIG. 5 is a flowchart illustrating the overall process of a fusion gene detection method according to the present invention.

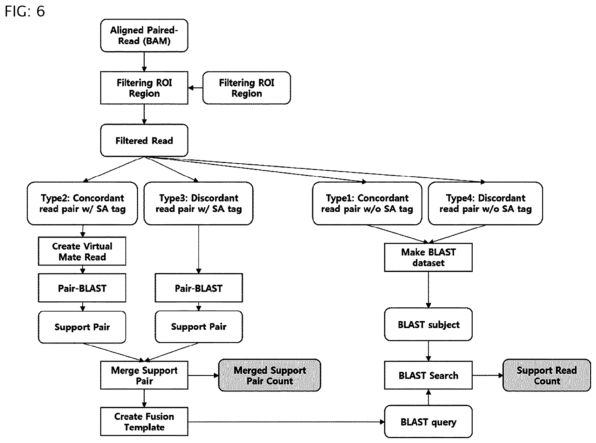

FIG. 6 is a flowchart illustrating each process of gene rearrangement according to an embodiment of the present invention.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

As used herein, the term "next-generation sequencing" or "NGS" refers to a sequencing method that determines the nucleotide sequence of one of proxies expanded with clones for an individual nucleic acid molecule in an individual nucleic acid molecule mode (e.g., in single-molecule sequencing) or in high-speed bulk mode (e.g., when sequencing 10, 100, 1000 or more molecules simultaneously). In one embodiment, the relative abundance of nucleic acid species in the library can be estimated by measuring, in the data generated by the sequencing experiments, the relative number of occurrences of cognate sequences thereof. Next-generation sequencing methods are known in the art and are described, for example, in [Metzker, M. (2010) Nature Biotechnology Reviews 11:31-46]. Next-generation sequencing can detect variants present in less than 5% of nucleic acids in a sample.

The next-generation sequencing process in the present invention can be divided into the following three steps.

(1) Capture of target

Next-generation sequencing can be used to sequence the whole genome, to sequence only exome regions (targeted sequencing), or to sequence only specific genes in order to find genes causative of diseases. Sequencing only exome regions or specific target genes is advantageous in terms of cost or efficiency. In addition, since variations of genes are often directly caused by diseases such as cancer, detecting the change in the nucleotide sequence in the exome region or the target gene may be effective in finding genes causative of diseases. In order to sequence only exomes or target genes, a library capable of capturing only the exomes or target genes is required.

The library mainly used for capturing exome regions includes SureSelect Human All Exon Kits (http://www.genomics.agilent.com), but is not limited thereto. SureSelect Human All Exon Kits are designed on the basis of exons of gene sets of the human genome defined by CCDS (consensus CDS, NCBI, EBI, UCSC and Wellcome trust Sanger Institute) and include an area corresponding to 1.22% of the human genome.

In order to capture only target genes, probes or baits specific to the certain target genes may be used.

(2) Large-capacity parallel DNA sequencing

Next-generation sequencing (NGS) has advantages of simultaneously identifying a greater amount of sequences more quickly at once than conventional capillary sequencing, and of omitting a process of amplifying the sample, thus avoiding experimental error occurring in this process.

NGS systems produced by three companies are mainly used. 454 GS FLX of Roche AG launched in 2004 was the first NGS instrument capable of performing sequencing using pyrosequencing and emulsion polymerase chain reactions and determining specific bases depending on the intensity of light emitted during the final stage of the experiment. When operated for 7 hours, the 454 GS FLX can identify a sequence of about 100Mb, which is much higher than a conventional ABI 3730 device, which can identify a sequence of 440kb within the same time.

The Illumina genome analyzer produced by Illumina, Inc. is based on the concept of sequencing by synthesis. After attaching single-stranded DNA fragments onto a glass plate, the fragments are polymerized and clustered. During this process, sequence analysis is performed while determining the type of bases attached to the DNA fragments to be tested. After operation for about four days, about 40 to 50 million fragments having a base length of 32 to 40 are produced.

The SOLiD (sequencing by oligo ligation) apparatus produced by Life Technologies Inc. is designed to perform sequencing using an emulsifier-polymerase chain reaction after attaching a DNA fragment to be tested to a 1 $\mu$m magnetic bead. Sequencing is carried out by repeatedly attaching 8-mer fragments to each other. The bases used for actual sequencing are positioned at the 4th and 5th 8-mer fragments. A fluorescent material is linked to the remainder behind them to mark the base that complementarily binds to the DNA fragment to be tested. By attaching all 8-mers five times in one binding cycle and performing the same operation five times, a sequence of DNA fragments consisting of a total of 25 bases can be identified. The SOLiD instrument is characterized by sequencing using two-base encoding. This method identifies the same region through double sequencing when determining the sequence of one base. Sequencing is performed while shifting the sequence by one base in one binding cycle toward the adaptor attached to the magnetic bead. This process has the advantage of eliminating errors that occur in sequencing experiments.

(3) Analysis of base sequence data

In order to find genes causative of diseases, it is necessary to investigate what changes have been made from the original gene sequence. Thus, an operation of comparing nucleotide data (sequence reads) of an individual (patient) with the reference genome is performed. This operation is called mapping. Differences between the individual sequence and the reference sequence are identified through mapping, appropriate selection criteria are set based on the differences, and only reliable sequence variant information is extracted (variant calling). This variation information is structural variation (SV) that includes single nucleotide variation (SNV), short indel, copy number variation (CNV), fusion genes and the like. Then, the nucleotide variation information is compared with the existing database to determine whether it is a known or newly discovered variation. Also, whether or not the variation will result in a change in amino acids and how it affects protein structure is predicted. This process is called "annotation". Information associated with extracted single-nucleotide sequence variations and short indel may be listed in the database so as to improve the quality of the information, or research to find variations causative of diseases can be conducted through studies integrated with the genome wild association study (GWAS).

However, a conventional method is capable of highly accurately extracting, as variation information for calling, variation information such as SNV, Indel or CNV, but has a disadvantage of low accuracy of the structural variation. In particular, according to the method, a method of extracting a structural variation, in particular, variation information of fusion genes, with high accuracy is developed.

The terms "cancer" and "tumor" are used interchangeably herein. These terms refer to the presence of cells possessing typical features of cancer-causing cells such as uncontrolled proliferation, immortality, the potential of metastasis, rapid growth and proliferation rates, and certain characteristic morphological features. Cancer cells are often in the form of tumors, but such cells may be present alone in an animal, or may be non-tumor cancer cells such as leukemia cells. These terms include solid tumors, soft-tissue tumors or metastatic lesions. As used herein, the term "cancer" includes both premalignant cancer and malignant cancer.

As used herein, the term "sample", "tissue sample", "cancer sample", "patient sample", "patient cell or tissue sample" or "specimen" refers to a collection of similar cells obtained from tissue or circulating cells of a subject or patient, respectively. Sources of the tissue sample include: solid tissue from fresh, frozen and/or preserved organs, tissue samples, biopsies or inhalations; blood or any blood ingredient; body fluids such as cerebrospinal fluid, amniotic fluid, peritoneal fluid or interstitial fluid; or cells from any point in pregnancy or development of a subject. The tissue sample may include compounds that are not naturally admixed with tissue in nature, such as preservatives, anticoagulants, buffers, fixatives, nutrients and antibiotics. In one embodiment, the sample is prepared as a frozen sample or as a formaldehyde- or paraformaldehyde-fixed paraffin-embedded (FFPE) tissue preparation. For example, the sample may be embedded in a matrix such as an FFPE block, or may be a frozen sample.

In one embodiment, the sample is a cancer sample, e.g., includes one or more precancerous or malignant cells. In certain embodiments, the sample, e.g., a tumor sample, is acquired from a solid tumor, soft-tissue tumor, or metastatic lesion. In other embodiments, the sample, e.g., a tumor sample, includes tissue or cells from a surgical resection. In other embodiments, the sample, e.g., a tumor sample, includes one or more circulating tumor cells (CTCs) (e.g., CTCs acquired from blood samples).

As used herein, the term "acquire" or "acquiring" refers to possessing a physical entity or value, such as a numerical value, by "directly acquiring" or "indirectly acquiring" a physical entity or value. "Indirectly acquiring" means performing a process to acquire a physical entity or value (e.g., performing a synthetic or analytical method). "Indirectly acquiring" refers to receiving a physical entity or value from another party or source (e.g., a third-party laboratory that directly acquired the physical entity or value).

Indirectly acquiring a physical entity involves performing a process involving a physical change from a physical material, for example, a starting material. Representative changes include performing chemical reactions involving forming physical entities from two or more starting materials, shearing or fragmenting materials, separating or purifying materials, combining two or more separate entities into a mixture, and breaking or forming a covalent or non-covalent bond. Indirectly acquiring a value includes performing a treatment involving a physical change from a sample or other material, for example, performing an analytical process involving a physical change from a material, for example, a sample, analyte or reagent (often referred to herein as "physical analysis"), performing an analytical method, e.g., a method including one or more of the following: separating or purifying a material, such as an analyte or fragment or other derivative thereof, from another material; combining an analyte or fragment or other derivative thereof with another material, such as a buffer, solvent or reactant; or changing the structure of an analyte or fragment or other derivative thereof, for example, by breaking or forming a covalent or non-covalent bond between the first and second atoms of the analyte; or changing the structure of a reagent or fragment or other derivative thereof, for example by breaking or forming a covalent or non-covalent bond between the first and second atoms of the reagent.

As used herein, the term "acquiring a sequence" or "acquiring a read" refers to possessing a nucleotide sequence or amino acid sequence by "directly acquiring" or "indirectly acquiring" the sequence or read. "Directly acquiring" a sequence or read refers to performing a process for acquiring the sequence (e.g., performing a synthetic or analytical method), for example, performing a sequencing method (e.g., a next-generation sequencing (NGS) method). "Indirectly acquiring" a sequence or read refers to receiving a sequence from another party or source (e.g., a third-party laboratory that directly acquired the sequence) or receiving information or knowledge of the sequence. The acquired sequence or read need not be a complete sequence, and acquiring information or knowledge to identify one or more of the alterations disclosed herein, for example, sequencing of at least one nucleotide or presence in a subject, constitutes acquiring a sequence.

Directly acquiring a sequence or read includes performing a process involving a physical change from a physical material, e.g., a starting material, such as a tissue or cell sample, e.g., a biopsy or an isolated nucleic acid (e.g., DNA or RNA) sample. Representative changes include shearing or fragmenting two or more materials, for example, starting materials, such as producing physical entities from genomic DNA fragments (e.g., separating nucleic acid samples from tissue); performing a chemical reaction including combining two or more separate entities into a mixture, and breaking or forming a covalent or non-covalent bond. Directly acquiring a value includes performing a process involving a physical change from the sample or other material as described above.

As used herein, the term "nucleic acid" or "polynucleotide" refers to a single- or double-stranded deoxyribonucleic acid (DNA) or ribonucleic acid (RNA) and polymers thereof. Unless specifically limited otherwise, the term includes nucleic acids containing known analogues of natural nucleotides that have binding properties similar to those of the reference nucleic acid and are metabolized in a manner similar to natural nucleotides. Unless otherwise stated, certain nucleic acid sequences also include not only clearly disclosed sequences but also implicitly conservatively modified variants thereof (e.g., degenerate codon substitutions), alleles, orthologs, SNPs and complementary sequences. Specifically, degenerate codon substitutions can be carried out by forming a sequence in which position 3 of one or more selected codons (or all codons) is substituted with a mixed base and/or a deoxyinosine residue (Batzer et al., Nucleic Acid Res.19:5081 (1991); Ohtsuka et al., J. Biol. Chem. 260:2605-2608 (1985); and Rossolini et al., Mol. Cell. Probes 8:91-98 (1994)). The term "nucleic acid" is used interchangeably with a gene, cDNA, mRNA, small non-coding RNA, micro RNA (miRNA), Piwi-interacting RNA and short hairpin RNA (shRNA) encoded by a gene or locus.

In the present invention, the bait can be used not only to capture a target gene but also to capture a specific region of the target genome in any region of the sample genome (e.g., 5'-UTR, intron, microsatellite region, centromere region or telomere region).

The bait in the present invention may be used as a combination of a variety of baits, but is not limited thereto, and preferably includes two or more of the following:

a) a first bait set for detecting a variation occurring at a frequency of about 5% or less, wherein the first bait set is capable of detecting a variation requiring a read depth of about 500X or more;

b) a second bait set for detecting a variation occurring at a frequency of about 10% or more, wherein the second bait set is capable of detecting a variation requiring a read depth of about 200X or more;

c) a third bait set for detecting drug-metabolism-related SNP, patient-specific (genomic fingerprint) SNP and/or loss of heterozygosity (LOH), wherein the third bait set is capable of detecting a variation requiring a read depth of 10 to 100X;

d) a fourth bait set for detecting a structural variation, wherein the fourth bait set is capable of detecting a variation requiring a read depth of 5 to 50X; and

e) a fifth bait set for detecting a copy number variation, wherein the fifth bait set is capable of detecting a variation requiring a read depth of 0.1 to 300X.

The values for the efficiency of bait selection in the present invention may be changed by one or more of the following: differential representation of different bait sets, differential overlap of bait subsets, differential bait variables, mixing of different bait sets, and/or the use of different types of bait sets. For example, the change in selection efficiency (e.g., relative sequence coverage of each bait set/target category) can be adjusted by changing one or more of the following:

(i) differential representation of different bait sets - bait sets designed to capture a given target (e.g., target member) may be included in more or less replicas to improve or reduce relative target coverage depths;

(ii) differential overlap of bait subsets - bait set designed to capture a given target (e.g., target member) include longer or shorter replicas between adjacent baits to improve or reduce relative target coverage depths;

(iii) differential bait variables - bait set designed to capture a given target (e.g., target member) include sequence modifications/shorter lengths to reduce capture efficiency and reduce relative target coverage depths;

(iv) mixing different bait sets - bait sets designed to capture different target sets can be mixed at different molar ratios to improve and reduce relative target coverage depths;

(v) use of different types of oligonucleotide bait sets - in certain embodiments, the bait sets may include the following:

(a) one or more chemically (e.g., non-enzymatically) synthesized (e.g., individually synthesized) baits,

(b) one or more baits synthesized in an array,

(c) one or more enzymatically produced baits, e.g., baits transcribed *in vitro;*

(d) any combination of (a), (b) and/or (c),

(e) one or more DNA oligonucleotides (e.g., naturally or non-naturally occurring DNA oligonucleotides),

(f) one or more RNA oligonucleotides (e.g., naturally or non-naturally occurring RNA oligonucleotides),

(g) a combination of (e) and (f), or

(h) any combination of those described above.

The combination of different oligonucleotides may be mixed in different ratios, for example, a ratio selected from 1:1, 1:2, 1:3, 1:4, 1:5, 1:10, 1:20, 1:50, 1:100, or 1:1000. In one embodiment, the ratio of chemically synthesized baits to alignment-produced baits is selected from 1:5, 1:10 or 1:20. DNA or RNA oligonucleotides may occur naturally or non-naturally.

In certain embodiments, the bait includes, for example, one or more non-naturally occurring nucleotides in order to increase the melting point. Representative non-naturally occurring oligonucleotides include modified DNA or RNA nucleotides. Representative modified nucleotides (e.g., modified RNA or DNA nucleotides) are modified by the following, which include, but are not limited to, locked nucleic acid (LNA), wherein the ribose moiety of the LNA nucleotide is an additional bridge linking the 2' oxygen to the 4' carbon; peptide nucleic acid (PNA), for example, PNA consisting of repeating N-(2-aminoethyl)-glycine units linked by peptide bonds; DNA or RNA oligonucleotides modified to capture low GC regions; bicyclic nucleic acid (BNA); crosslinked oligonucleotides; modified 5-methyl deoxycytidine; and 2,6-diaminopurine. Other modified DNA and RNA nucleotides are known in the art.

In certain embodiments, substantially uniform or equivalent coverage of the target sequence (e.g., target member) is obtained. For example, within each bait set/target category, the uniformity of coverage can be optimized by modifying the bait variable using, for example, one or more of the following:

(i) an increase/decrease in bait expression or duplication may be used to improve/reduce the coverage of a target (e.g., a target member) that is covered under or over another target within the same category;

(ii) a targeted area is expanded with a bait set covering adjacent sequences (e.g., fewer GC-rich adjacent sequences) with regard to a low coverage that makes it difficult to capture target sequences (e.g., high GC content sequences);

(iii) modification of the bait sequence may be designed to reduce the secondary structure of the bait and improve selection efficiency thereof;

(iv) change in bait length may be used to realize identical melt hybridization kinetics of different baits within the same category. The bait length may be modified directly (by generating baits having various lengths) or indirectly (by generating baits having constant length and replacing the bait ends with arbitrary sequences);
Modifying baits of different orientations with regard to the same target region (i.e., front and back strands) may cause different binding efficiencies. A bait set that has one of the orientations that provide the optimal coverage for each target may be selected;

(vi) Modification in the amount of a binding entity, for example, a capture tag (e.g., biotin) present in each bait, may affect the binding efficiency thereof. An increasing/decrease in the tag level of the bait targeting a specific target may be used to improve or reduce relative target coverages;

(vii) modification of the nucleotide type used for different baits may be changed to affect the binding affinity of the target, and can improve or reduce relative target coverages; or

(viii) For example, the modified oligonucleotide baits having more stable base pairs may be used such that the melt hybridization kinetics between regions of low or normal GC contents are equal for high GC content.

For example, different types of oligonucleotide bait sets may be used.

In one embodiment, the value for efficiency of selection is modified by using different types of bait oligonucleotides to include a preselected target region. For example, a first bait set (e.g., an array-based bait set including 10,000 to 50,000 RNA or DNA baits) may be used to cover a large target area (e.g., a total target area of 1 to 2 MB). The first

bait set may be spiked with a second bait set (for example, an individually synthesized RNA or DNA bait set including less than 5,000 baits) to cover a pre-selected target region (for example, selected subgenomic intervals of interest, for example, a target area of 250 kb or less) and/or a region of higher secondary structure, for example, higher GC content. The selected subgenomic intervals of interest may correspond to one or more of the genes described herein or gene products or fragments thereof. The second bait set may include about 1 to 5,000, 2 to 5,000, 3 to 5,000, 10 to 5,000, 100 to 5,000, 500 to 5,000, 100 to 5,000, 1000 to 5,000, or 2,000 to 5,000 baits depending on the desired bait overlap. In other embodiments, the second bait set may include selected oligo baits spiked into the first bait set (e.g., less than 400, 200, 100, 50, 40, 30, 20, 10, 5, 4, 3, 2 or 1 bait). The second bait set may be mixed at any ratio of individual oligo baits. For example, the second bait set may include individual baits present at a 1:1 equimolar ratio. Alternatively, the second bait set may include individual baits present at different ratio (for example, 1:5, 1:10, 1:20), for example, to optimize capture of certain targets (e.g., certain targets may have 5-10X of the second bait compared to other targets).

In other embodiments, the efficiency of selection is adjusted by leveling the efficiency of individual baits within a group (for example, a first, second or third plurality of baits) by adjusting the relative abundance of the baits or the density of the binding entity (for example, the hapten or affinity tag density) with regard to differential sequence capture efficiency observed when using an equimolar mix of baits, and then introducing a differential excess of internally leveled group 1 to the overall bait mix compared to internally leveled group 2.

In an embodiment, the method includes the use of a plurality of bait sets that includes a bait set that selects a nucleic acid molecule including a target sequence from a tumor member, for example, a tumor cell. The tumor member may be any nucleotide sequence present in a tumor cell, for example, a mutated sequence, a wild-type sequence, a PGx, a reference or an intron nucleotide sequence, as described herein, that is present in a tumor or cancer cell. In one embodiment, the tumor member includes an alteration (for example, at least one mutation) appearing at a low frequency, for example, includes an alteration in about 5% or less of the cell from the tumor sample in the genome thereof. In another embodiment, the tumor member includes an alteration (for example, at least one mutation) appearing at a frequency of about 10% of the cell from the tumor sample.

In another embodiment, the tumor member includes a target sequence from a PGx gene or gene product, an intron sequence, for example, an intron sequence described herein, and a reference sequence present in a tumor cell.

In another aspect, the present invention features a bait set described herein and combinations of individual bait sets described herein, for example, combinations described herein. The bait set(s) may be a part of a kit which may optionally include instructions, standards, buffers, enzymes or other reagents.

Regarding the term "paired-end read" as used herein, the term "paired end" refers to two ends of the same DNA molecule. When one end is sequenced and then turned over and the other end is sequenced, these two ends, the base sequence of which is identified, are called "paired-end reads". For example, Illumina sequencing generates a read of about 500 bps and reads a nucleotide sequence 75 bps long at each end of the read. At this time, the reading directions of the two reads (the first read and the second read) are 3' and 5', which are opposite each other, respectively, and mutually become paired-end reads.

As used herein, the terms "first read" and "second read" refer to a first read in the 5' direction and a second read in the 3' direction, acquired through paired-end read sequencing.

As herein used, the term "soft-clip", "soft-clip segment" or "soft-clipped read" refer to a read in which only a portion of the read acquired through NGS is mapped to the reference genome and the rest thereof is not mapped thereto.

As herein used, the term "discordant read pair" refers to a pair of reads (a first read and a second read) acquired by paired-end read sequencing, which are not mapped on the same reference gene, but are mapped at different positions or on different chromosomes.

As herein used, the term "concordant read pair" refers to a pair of reads (first read and second read) acquired by paired-end read sequencing, which are mapped to the same gene, but have information in which the soft-clip fragment portion of the read is mapped to different genes.

As herein used, the term "supporting pair count" means that, when the number of read pairs matching both the first gene and the second gene of the fusion gene is one or more, the number is increased by one. In this case, the number of read pairs may be two or more, regardless of whether the read pair is a discordant read pair or a concordant read pair.

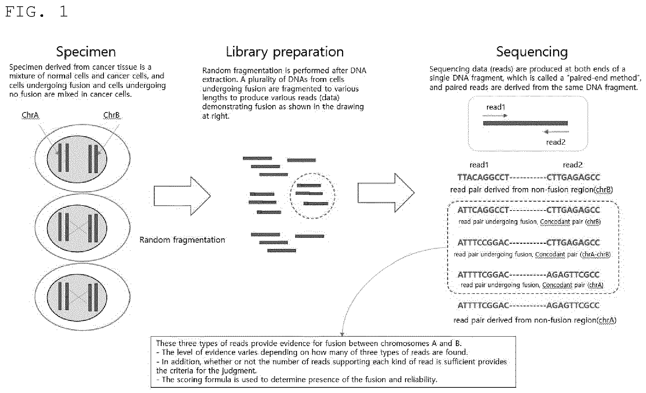

In the present invention, the nucleic acid was extracted from the cancer sample, the read was acquired through NGS, and then whether gene rearrangement could be detected using both the discordant read pair and the concordant read pair was determined (FIG. 1).

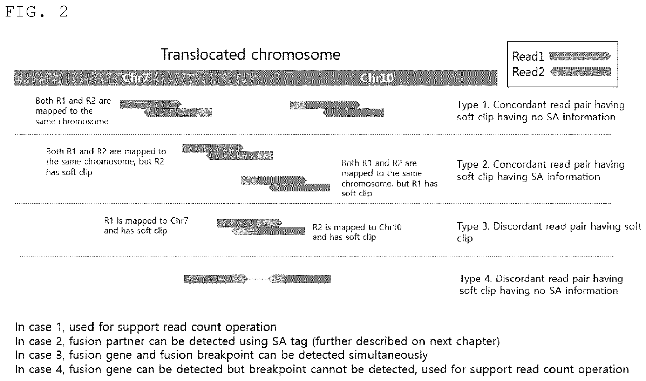

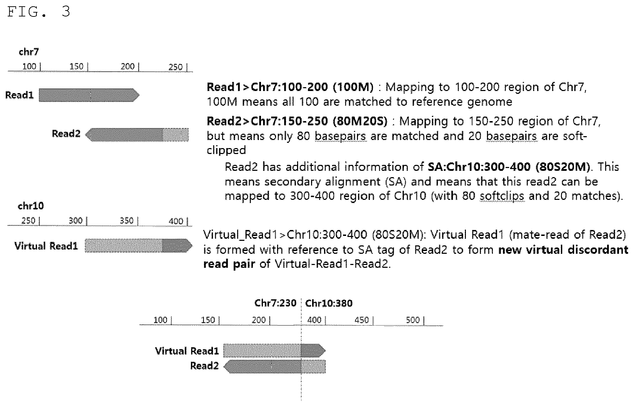

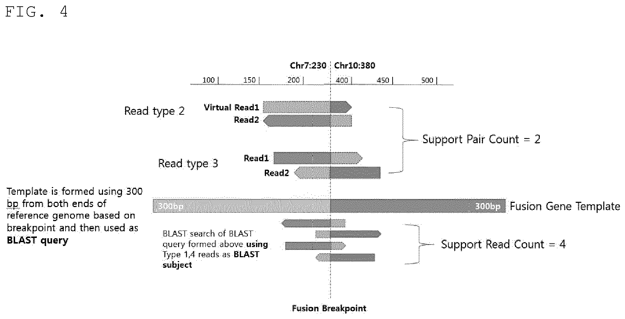

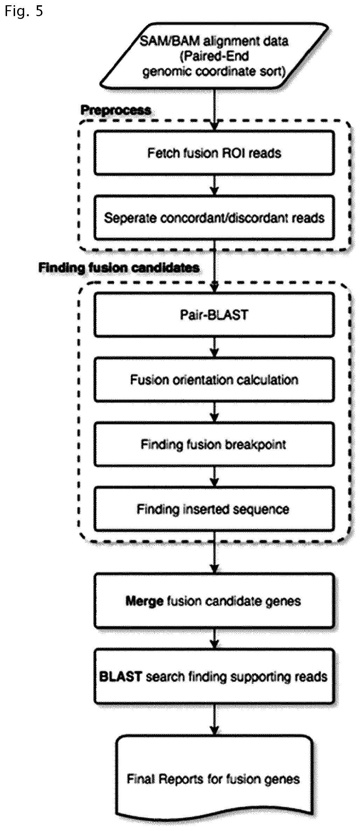

That is, in one embodiment of the present invention, a nucleic acid was extracted from a FFPE sample acquired from a lung cancer tissue sample, the read was acquired through NGS and arranged, and then fusion gene candidate reads were extracted to separate discordant read pairs and concordant read pairs (FIG. 2). Then, a fusion gene candidate group was derived from the read pairs through pair-blast search to determine a supporting pair count (FIG. 3). Among the acquire reads, an unextracted read was matched to a fusion gene template produced from the fusion gene candidate group to determine a supporting read count, and then fusion genes were finally detected in consideration of the supporting pair count and the supporting read count (FIG. 4). The result was compared with a conventional well-known program, FACTERA (Fusion gene And Chromosomal Translocation Enumeration and Recovery Algorithm, Aaron M. et al., 2014) . The result showed that a fusion gene that could be detected by a well-known program could be detected by the method of the present invention (FIG. 5, Table 1).

In one aspect, the present invention is directed to a method of detecting a gene rearrangement in a sample including: (a) acquiring a library including a plurality of nucleic acid molecules from a sample of a subject; (b) bringing the library into contact with a plurality of bait sets to enrich the library with respect to a preselected sequence to provide a selected nucleic acid molecule and thereby provide a library catch; (c) acquiring reads from the nucleic acid molecules of the library catch through next-generation sequencing; (d) arranging the reads using an alignment method; and (e) analyzing the arranged reads to detecting a gene rearrangement, wherein the analysis method includes extracting the arranged reads to analyze sequence similarity.

In the present invention, the cancer is selected from the group consisting of non-Hodgkin lymphoma, Hodgkin lymphoma, acute-myeloid leukemia, acute lymphoid leukemia, multiple myeloma, head and neck cancer, lung cancer, glioblastoma, colon/rectal cancer, pancreatic cancer, breast cancer, ovarian cancer, melanoma, prostate cancer, kidney cancer and mesothelioma, but is not limited thereto.

In the present invention, the sample includes: one or more premalignant or malignant cells; cells selected from solid tumors, soft tissue tumors or metastatic lesions; tissue or cells from surgical resections; histologically normal tissue; at least one blood tumor cell (CTC); and blood samples from the same subject having or at risk of developing a normal adjacent tumor (NAT) and a tumor, and is preferably a FFPE sample, but is not limited thereto.

In the present invention, the gene rearrangement means any variation in which the position of the nucleotide sequence is changed relative to the normal genome, regardless of the type thereof, and may be selected from the group consisting of gene fusion, translocation, inversion and deletion, but is not limited thereto.

In the present invention, the method is applied to DNA NGS analysis or RNA NGS analysis, but is not limited thereto, and it will be obvious to those skilled in the art that the method is applicable to all methods capable of analyzing gene rearrangement by NGS. (content added)

In the present invention, the arrangement of the reads in step (d) may be performed through any method using a program capable of arranging the reads acquired by next-generation sequencing (NGS) in the genome coordinates, but is preferably performed using BWA (Burrows-Wheeler Aligner), without being limited thereto.

In the present invention, when using BWA, a mark shorter split hits as secondary (-M) option for adding a secondary alignment tag in order to add information about a concordant read pair may be used, but the present invention is not limited thereto.

In the present invention, the reference genome for performing arrangement of reads in step (d) may be characterized by using the complete (entire) genome of normal cells, for example, hg19, but is not limited thereto.

In the present invention, the format of the read file arranged in step (d) may be a BAM/SAM file, but is not limited thereto.

In the present invention, the candidate read extraction in step (d) may be performed by filtering the read acquired in step (a) with information of the region of interest, but is not limited thereto.

In the present invention, the region of interest may include information on the location of a known fusion gene and information on a target gene region, wherein information of the region of interest includes chromosome information and information of start and end positions on the chromosome.

In the present invention, the information of the region of interest may include, but is not limited to, the contents disclosed in Table 1 below.

[Table 1]

| Information of region of interest | | | |
|---|---|---|---|
| Chromosome | Start Position | Stop Position | Gene Name |
| chr1 | 156843424 | 156843751 | NTRK1_10 |
| chr1 | 156843751 | 156844174 | NTRK1_i_01 |
| chr1 | 156844174 | 156844192 | NTRK1_11 |
| chr1 | 156844192 | 156844362 | NTRK1_i_02 |
| chr1 | 156844362 | 156844418 | NTRK1_12 |
| chr1 | 156844418 | 156844607 | NTRK1_i_03 |

(continued)

| Information of region of interest | | | |
|---|---|---|---|
| Chromosome | Start Position | Stop Position | Gene Name |
| chr1 | 156844697 | 156844800 | NTRK1_13 |
| chr1 | 156844800 | 156845311 | NTRK1_i_04 |
| chr1 | 156845311 | 156845458 | NTRK1_14 |
| chr1 | 156845458 | 156845871 | NTRK1_i_05 |
| chr1 | 156845871 | 156846002 | NTRK1_15 |
| chr2 | 29446207 | 29446394 | ALK_10 |
| chr2 | 29446394 | 29446326 | ALK_i_01 |
| chr2 | 29448326 | 29448431 | ALK_11 |
| chr2 | 29448431 | 29449787 | ALK_i_02 |
| chr2 | 29449787 | 29449940 | ALK_12 |
| chr2 | 29449940 | 29450439 | ALK_i_03 |
| chr2 | 29450439 | 29450538 | ALK_13 |
| chr2 | 29450538 | 29451749 | ALK_i_04 |
| chr2 | 29451749 | 29151932 | ALK_14 |
| chr4 | 1808272 | 1808410 | PGFR3_16 |
| chr4 | 1808410 | 1808555 | FGFR3_i_01 |
| chr4 | 1808655 | 1808661 | FGFR_17 |
| chr4 | 1808661 | 1808842 | PGFR3_i_03 |
| chr4 | 1808842 | 1808989 | FGFR3_18 |
| chr6 | 117641030 | 117641193 | ROS1_08 |
| chr6 | 117641193 | 117642421 | ROS1_i_01 |
| chr6 | 117642421 | 117642557 | ROS1_09 |
| chr6 | 117642557 | 117645494 | ROS1_i_02 |
| chr6 | 117645494 | 117645578 | ROS1_10 |
| chr6 | 117645578 | 117647386 | ROS1_i_03 |
| chr6 | 117647386 | 117647577 | ROS1_11 |
| chr6 | 117647577 | 117650491 | ROS1_i_04 |
| chr6 | 117650491 | 117650609 | ROS1_12 |
| chr6 | 117650609 | 137658334 | ROS1_i_05 |
| chr6 | 117658334 | 117658503 | ROS1_13 |
| chr7 | 55259411 | 55259567 | EGFR_23 |
| chr7 | 55259567 | 55260458 | EGFR_i_23 |
| chr7 | 55260458 | 55260534 | EGFR_21 |
| chr7 | 55260534 | 55266409 | BGFR_i_24 |
| chr7 | 55206409 | 55266556 | EGFR_25 |
| chr7 | 55266556 | 55268008 | EGFR_i_25 |
| chr7 | 55268006 | 55268106 | EGFR_26 |

(continued)

| Information of region of interest | | | |
|---|---|---|---|
| Chromosome | Start Position | Stop Position | Gene Name |
| chr8 | 38283639 | 38283763 | FGFR1_13 |
| chr8 | 38283763 | 38285438 | FGFR1_i_01 |
| chr8 | 38285438 | 38285611 | FGFR1_14 |
| chr10 | 43609003 | 43609123 | REX_10 |
| chr10 | 43609123 | 43609927 | RET_i_01 |
| chr10 | 43609927 | 43610184 | RET_11 |
| chr10 | 43610184 | 43612031 | RET_i_02 |
| chr10 | 43612031 | 43612170 | RET_12 |
| chr10 | 43612179 | 43613820 | RET_i_03 |
| chr10 | 43613820 | 43613928 | RET_13 |
| chr10 | 123239094 | 123239184 | PGFR2_01 |
| chr10 | 123239134 | 123239370 | FGFR2_i_01 |
| chr10 | 123239370 | 123239535 | FGFR2_02 |
| chr10 | 123239535 | 123241685 | FGFR2_i_02 |
| chr10 | 123241685 | 123241691 | FGFR2_03 |
| chr10 | 123241691 | 323243211 | FGFR2_i_03 |
| chr10 | 123243211 | 123243317 | FGFR2_04 |
| chr1 | 114935399 | 115053781 | TRIM33 |
| chr1 | 154127780 | 154164611 | TPM3 |
| chr1 | 156062369 | 156109880 | LMNA |
| chr1 | 156611740 | 156629324 | BCAN |
| chr1 | 204797783 | 204991950 | NFASC |
| chr1 | 205626979 | 205649630 | SLC15A3 |
| chr10 | 32297938 | 32345371 | KIF5B |
| chr10 | 51565108 | 51500734 | NCOA4 |
| chr10 | 60272774 | 60591194 | BICC1 |
| chr10 | 61548505 | 61666414 | CCDO6 |
| chr10 | 75757836 | 75879918 | VCL |
| chr10 | 115438921 | 115490668 | CASP7 |
| chr10 | 110042888 | 118886097 | KIAA1598 |
| chr11 | 3022152 | 3078681 | CARS |
| chr11 | 62623484 | 62656355 | SLC3A2 |
| chr12 | 1100404 | 1605099 | ERC1 |
| chr12 | 27677045 | 27848497 | PPFIBP1 |
| chr12 | 59265937 | 59314319 | LRIG3 |
| chr12 | 122755981 | 122907179 | CLIP1 |
| chr12 | 122956146 | 122985543 | ZCCHC8 |

(continued)

| Information of region of interest | | | |
|---|---|---|---|
| Chromosome | Start Position | Stop Position | Gene Name |
| chr14 | 56046925 | 56151302 | KTN1 |
| chr14 | 93260576 | 93306308 | GOLGA5 |
| chr14 | 104095525 | 104167888 | KLC1 |
| chr15 | 40987327 | 41024356 | RAD51 |
| chr15 | 52599480 | 52821247 | MYO5A |
| chr17 | 7571720 | 7590868 | TP53 |
| chr17 | 16945790 | 17095962 | MPRIP |
| chr17 | 57697050 | 57774317 | CLTC |
| chr17 | 66507921 | 66547457 | PRKAR1A |
| chr18 | 59854506 | 59974355 | KIAA1468 |
| chr19 | 16178317 | 16213815 | TPM4 |
| chr2 | 24252206 | 24270296 | C2orf44 |
| chr2 | 37064841 | 37193673 | STRN |
| chr2 | 42396490 | 42559688 | EML4 |
| chr2 | 54683454 | 54898583 | SPTBN1 |
| chr2 | 74588281 | 74619214 | DCTN1 |
| chr2 | 100162326 | 100759037 | AFF3 |
| chr2 | 109335902 | 109402267 | RANBP2 |
| chr2 | 216176679 | 216214496 | ATIC |
| chr2 | 216225177 | 216300890 | FN1 |
| chr20 | 43953928 | 43977064 | SDC4 |
| chr22 | 19166986 | 19279247 | CMTCL1 |
| chr3 | 100428128 | 100467811 | TFG |
| chr4 | 1723217 | 1746905 | TACC3 |
| chr4 | 25656853 | 25680735 | SLC34A2 |
| chr4 | 83739814 | 83812419 | SEC31A |
| chr5 | 149781200 | 149792543 | CD74 |
| chr5 | 159502889 | 159546452 | PWWP2A |
| chr5 | 170814852 | 170837888 | NPM1 |
| chr5 | 179233388 | 179266078 | SOSTM1 |
| chr6 | 28870779 | 28891768 | TRIM27 |
| chr6 | 29910247 | 29913661 | HLA-A |
| chr6 | 117881432 | 117923705 | GOPC |
| chr6 | 159186773 | 159240456 | EZR |
| chr7 | 44915892 | 44924960 | PURB |
| chr7 | 75162619 | 15368290 | HIP1 |
| chr7 | 97920962 | 98030427 | BAIAP2L1 |

(continued)

| Information of region of interest | | | |
|---|---|---|---|
| Chromosome | Start Position | Stop Position | Gene Name |
| chr7 | 101459184 | 101927250 | CUX1 |
| chr7 | 138145079 | 138270333 | TRIM24 |
| chr8 | 17780364 | 17887457 | PCM1 |
| chr8 | 22462145 | 22477984 | KIAA1967 |
| chr8 | 37553301 | 37566396 | ZNF703 |
| chr8 | 37593743 | 37615319 | ERLIN2 |
| chr8 | 38034106 | 38070819 | BAG4 |
| chr8 | 42752033 | 42885682 | HOOK3 |
| chr9 | 125703288 | 125867147 | RABGAP1 |
| chrX | 13733549 | 13787480 | OFD1 |
| chrX | 64808257 | 64961793 | MSN |

In the present invention, step (e) may include extracting the reads and then separating the reads into discordant read pairs and concordant read pairs.

In the present invention, the separating into discordant read pairs and the concordant read pairs may be carried out by matching the reference gene (RefGene) information to the reads.

In the present invention, any reference genome may be used as long as it has genome information capable of determining whether the read pair (first read and second read) acquired by paired-end read sequencing is discordant read pairs or concordant read pairs, but RefGene information derived from the USCS genome database (http://hgdownload.cse.ucsc.edu/goldenPath/hg19/bigZips/h g19.2bit) is preferably used.

In the present invention, the discordant read pair may or may not have a soft clip (FIG. 2, type 3, 4), and the concordant read pair has a soft clip, but does not have SA information, or has SA (FIG. 2, type 1, 2).

In the present invention, the method further, after separation of the discordant read pairs in step (e), includes finding a matching region of the second read that forms a pair using a soft-clip segment portion of the first read as a query, or finding a matching region of the first read that forms a pair using a soft-clip segment portion of the second read as a query.

In the present invention, the method further includes, after the separation of the concordant read pairs in step (e), finding a matching region of the second read that forms a pair using a soft-clip segment portion of the first read as a query, or finding a matching region of the first read that forms a pair using a soft-clip segment portion of the second read as a query, while assuming, as a virtual second read, the secondary mapping region obtained by determining whether or not there is mapping to other genes with reference to the secondary alignment tag information of the first read (FIG. 3) .

In the present invention, a method of finding a matching region of each read after separating the concordant read pair and the discordant read pair in step (e) may be broadly referred to as a "pair-blast search".

In the present invention, in the step of finding a matching region of the discordant read pair, when a matching region is found, a read including the matching region is derived as a gene rearrangement candidate group to determine a supporting pair count.

In the present invention, in the step of finding a matching region of the concordant read pair, when a matching region is found, a read including the matching region is derived as a gene rearrangement candidate group to determine a supporting pair count.

In the present invention, the supporting pair count may be determined by integrating the results determined from the discordant read pair and the concordant read pair.

In the present invention, the step of integrating the supporting pair count may include increasing the supporting pair count when the supporting pair count is determined even for the discordant read pair, and the supporting pair count is determined even for the concordant read pair. Although the type of the gene rearrangement is identical, when the position thereof is different, it may be determined to be different.

In the present invention, the step (e) may further include arranging the reads not extracted as candidates for gene rearrangement for further analysis.

In the present invention, the step (e) further includes producing a gene rearrangement template (FIG. 4, fusion gene template) based on the read information derived as the gene rearrangement candidate group.

In the present invention, the gene rearrangement template is a base sequence on the reference genome including 300 bp to 500 bp in the 5' direction and 300 bp to 500 bp in the 3' direction from the gene rearrangement position (for example, the breakpoint of the fusion gene when the gene rearrangement is a fusion gene), but is not limited thereto.

In the present invention, analyzing the sequence similarity in step (e) may further include comparing the unextracted reads for analysis as the gene rearrangement template and the gene rearrangement candidate group to determine a supporting read count.

In the present invention, the supporting read count is determined as the number of reads that are mapped while passing the breakpoint of the gene rearrangement in the gene rearrangement template after performing blast using the arranged reads as blastdb and using the gene rearrangement template as a query.

In the present invention, the unextracted read for analysis as the gene rearrangement candidate group may be a read present within 500 bp in the 5' direction and the 3' direction from the position of gene rearrangement candidate group, but is not limited thereto.

In the present invention, the unextracted read for analysis as the gene rearrangement candidate group may include a soft-clip segment.

In the present invention, the step of detecting the gene rearrangement may include determining, as a gene rearrangement, when the supporting read count is 5 or more.

In the present invention, the step of detecting the gene rearrangement may further include a reference value having two or more supporting pair counts, but is not limited thereto.

In the present invention, the supporting read count and supporting pair count for detecting the gene rearrangement may be determined by the following Equation:

$$\text{Equation 1: Supporting Pair Score = Discordant Supporting Pair Count + Concordant Supporting Pair Count}$$

$$\text{Equation 2: Supporting Read Score = Read1 Supporting Read Count + Read2 Supporting Read Count}$$

$$\text{Equation 2: Cutoff: Supporting Pair Score} >= 2 \text{ AND Supporting Read Score} >= 5$$

In another embodiment of the present invention, the read obtained through NGS from a FFPE sample of a lung cancer patient is arranged in the HG19 reference genome using the -M option of BWA, and then the read is extracted based on the information on the region of interest and matched to the genome information of the UCSC genome database to separate concordant read pairs and discordant read pairs. Then, in the case of the discordant read pair, the soft-clip fragments of the first read and the second read are matched to each other to find a matching part, and in the case of the concordant read pair, a virtual second read is produced and matched to the first read to find a matching part and determine the same as a supporting pair count. Then, a gene rearrangement template is produced using the gene rearrangement candidate group determined in the step, and the read not extracted in the step is matched to a gene rearrangement template to determine a supporting read count, and when the supporting read count is more than one in each of the first and second reads, a computer system that determines the supporting read count as a gene rearrangement is designed and tested. The result showed that it is possible to find a gene rearrangement that cannot be found using a conventional published program.

In another aspect, the present invention is directed to a computer system including a computer-readable medium encoded with a plurality of instructions for controlling a computing system to perform a method of detecting a gene rearrangement using next-generation sequencing (NGS), wherein the method includes: (a) acquiring a library including a plurality of nucleic acid molecules from a sample of a subject; (b) bringing the library into contact with a plurality of bait sets to enrich the library with respect to a preselected sequence to provide a selected nucleic acid molecule and thereby provide a library catch; (c) acquiring reads from the nucleic acid molecules of the library catch through next-generation sequencing; (d) arranging the reads using an alignment method; and (e) analyzing the arranged reads to detecting a gene rearrangement, wherein the analysis method includes extracting the arranged reads to analyze sequence similarity.

Hereinafter, the present invention will be described in more detail with reference to examples. However, it will be obvious to those skilled in the art that these examples are provided only for illustration of the present invention and should not be construed as limiting the scope of the present invention.

Example 1: Step of performing next-generation sequencing from lung cancer sample

DNA was extracted from a FFPE sample acquired from a lung cancer tissue sample having known fusion genes present therein through fluorescence *in-situ* hybridization (FISH) to produce a library, and NGS reads were acquired using Illumina's MiSeq.

Baits were designed to include all of the positions on the chromosome including the information on the region of interest in Table 1.

Example 2: Arrangement of sequenced sequences in reference genome and read sorting

The reads acquired in Example 1 were arranged with BWA in the Hg19 reference genome, and the analysis was performed by adding the option (-M) to add a secondary alignment tag in the arrangement program (BWA) for analysis of concordant read pairs.

The discordant and concordant read pairs were separated by inputting reads based on UCSC RefGene information (HCSC hg 19), and then the filtered reads were arranged in ascending order to make it easier to sequentially extract first and second reads.

Example 3: Pair-blast search and determination of supporting pair counts

In the case of the discordant read pair sorted (separated) in Example 2, the soft-clip segment portion of the first read (read1) was extracted to form a query, and the matching segment portion of the second read (read2), constituting a mate, is used as a subject to perform blastn search local alignment. The strands of reads aligned through this process were identified to determine the direction of read1 (gene1) and read2 (gene2), and a blastn search was performed in the same manner as above using the soft-clip segment of read2 as a query and using a matching segment part of read1 as a subject.

As a result, since match and mismatch information at the nucleotide level can be obtained, micro-homology sequences present in both fusion genes can be identified, and externally inserted sequences can be identified.

In the case of concordant read pairs, since read1 and read2 were mapped to the same gene, it was identified again whether or not they are secondarily mapped to genes other than read2 with reference to SA (secondary alignment) tag information, having additional information in read1. Sequencing was performed in the same manner as in the discordant read pair, assuming that the second mapping region is virtual read2.

The result showed that the fusion break point can be determined with the nucleotide base-pair resolution using fusion gene orientation, micro-homology and inserted-sequence information (Table 2) based on the following criteria: when integrating the fusion gene candidate groups derived from discordant and concordant read pairs, respectively, and determining supporting pair counts, in the case where a fusion gene candidate group is simultaneously determined from respective read pairs, a fusion gene, in which the supporting pair count is increased but the breakpoint differs by more than the number of micro-homology sequences, although the type of gene is identical, is not integrated into one fusion gene, but is recorded as another fusion gene.

[Table 2]

(Partial) Result until supporting pair count is determined

| fusion gene | fusion orient | gene1 | gene1 transc ript | gene1 strand | gene1 c hr | gene1 brea kpoint | gene1 orient | gene2 | gene2 transcri pt | gene2 strand | gene2 c hr | gene2 bre akpoint | gene2 orient | support pair cnt | new fusion gene | gene1 que ry size | gene2 que ry size | micro ho mology | discordan t pair cnt |
|---|---|---|---|---|---|---|---|---|---|---|---|---|---|---|---|---|---|---|---|
| SLC34A2>>R OS1 | >> | SLC34A 2 | NM_006424.2 | + | chr4 | 25679213 | > | ROS1 | NM_002944.2 | - | chr6 | 117656511 | > | 10 | SLC34A2>>ROS1 | 300 | 301 | 0 | 0 |
| EGFR<>TPK1 | <> | EGFR | NM_005228.3 | + | chr7 | 55266908 | < | TPK1 | NM_022445.3 | + | chr7 | 144478980 | > | 4 | EGFR<>TPK1 | 300 | 297 | 4 | 0 |
| FGFR1><PGB D5 | >< | FGFR1 | NM_015850.3 | - | chr8 | 38285389 | > | PGBD5 | NM_00125831 1.1 | - | chr1 | 230468742 | < | 4 | FGFR1><PGBD5 | 300 | 288 | 13 | 0 |
| ULK4>>RET | >> | ULK4 | NM_017886.2 | - | chr3 | 41561725 | > | RET | NM_020975.4 | + | chr10 | 43611745 | > | 3 | ULK4>>RET | 300 | 301 | 0 | 0 |
| ACOT7>>AFF 3 | >> | ACOT7 | NM_007274.3 | - | chr1 | 6408272 | > | AFF3 | NM_002285.2 | - | chr2 | 100632991 | > | 2 | ACOT7>>AFF3 | 301 | 301 | 1 | 0 |
| AFF3<>PARP4 | <> | AFF3 | NM_002285.2 | - | chr2 | 100477189 | < | PARP4 | NM_006437.3 | - | chr13 | 25062673 | > | 2 | AFF3<>PARP4 | 300 | 268 | 33 | 0 |
| AFF3<>WISP2 | <> | AFF3 | NM_002285.2 | - | chr2 | 100632570 | < | WISP2 | NM_003881.2 | + | chr20 | 43351474 | > | 2 | AFF3<>WISP2 | 300 | 301 | 0 | 1 |

Example 4: Determination of supporting read count

The remaining reads excluding the reads extracted in Example 2 were mapped to the fusion gene template by BLAST search to determine a supporting read count. First, the remaining reads excluding the reads used for the analysis of Example 3 were extracted to form a blastdb, and 300bp were extracted in each of the 5' direction and the 3' direction, based on the fusion gene candidate group obtained in Example 3, to produce a fusion gene template. Then, blastn search was performed using the fusion gene template as a query.

In the above process, instead of extracting all of the remaining reads, only the reads present within 500 base pairs of the fusion gene breakpoint in 5' and 3' directions were extracted, and only the reads having soft-clip segments were extracted and used for analysis. The number of reads mapped while passing through the fusion breakpoint of the fusion gene template was filtered and recorded as a fusion gene supporting read count.

The result showed that the supporting read counts were determined in the first and second reads, respectively.

[Table 3]

(Partial) result until supporting pair count is determined

| fusion_gene | fusion_orient | gene1 | gene1_tx | gene1_strand | gene1_chr | gene1_bp | gene1_orient | inserted_seq | gene2 | gene2_tx | gene2_strand | gene2_chr | gene2_bp | gene2_orient | Supporting Pair Count | new_fusion_gene | Support Read Count (BLAST) |
|---|---|---|---|---|---|---|---|---|---|---|---|---|---|---|---|---|---|
| HIP1>>ALK | >> | HIP1 | NM_0053 38.5 | - | chr7 | 75172169 | > |  | ALK | NM_0043 04.4 | - | chr2 | 29446393 | > | 130 | HIP1>>ALK | 224 |
| NTRK3>>FN1 | >> | NTRK3 | NM_0025 30.3 | - | chr15 | 88472629 | > | G | FN1 | NM_0020 26.2 | - | chr2 | 21624566 | > | 5 | NTRK3>>FN1 | 10 |
| MET>>MPRIP | >> | MET | NM_0002 45.2 | + | chr7 | 11634023 1 | > |  | MPRIP | NM_0151 34.3 | + | chr17 | 17035425 | > | 5 | MET>>MPRIP | 5 |
| FBXL2>>ROS1 | >> | FBXL2 | NM_0121 57.3 | + | chr3 | 33358244 | > | A | ROS1 | NM_0029 44.2 | - | chr6 | 11764758 6 | > | 4 | FBXL2>>ROS1 | 8 |
| EGFR>>SLC34A2 | >> | EGFR | NM_0052 28.3 | + | chr7 | 55266557 | > | CTGGATGAT AGACGCAG ATAGTCGCC | SLC34A2 | NM_0064 24.2 | + | chr4 | 25679663 | > | 3 | EGFR>>SLC34A2 | 500 |
| KIAA1967>>JAK1 | >> | KIAA1967 | NM_0211 74.5 | + | chr8 | 22476766 | > | ATAAATG | JAK1 | NM_0022 27.2 | - | chr1 | 65349161 | > | 3 | KIAA1967>>JAK1 | 476 |
| KDM6A>>TP53 | >> | KDM6A | NM_0211 40.2 | + | chrX | 44790819 | > | TGCTCAGAT AGCGAT | TP53 | NM_0011 26113.2 | - | chr17 | 7578290 | > | 3 | KDM6A>>TP53 | 222 |
| EGFR>>YWHAG | >> | EGFR | NM_0052 28.3 | + | chr7 | 55259563 | > | G | YWHAG | NM_0124 79.3 | - | chr7 | 75958412 | > | 3 | EGFR>>YWHAG | 6 |
| FAM20A><ROS1 | >< | FAM20A | NM_0175 65.3 | + | chr17 | 66539615 | > | G | ROS1 | NM_0029 44.2 | - | chr6 | 11768723 9 | < | 3 | FAM20A><ROS1 | 6 |
| SLC34A2>>EGFR | >> | SLC34A2 | NM_0064 24.2 | + | chr4 | 25658933 | > | AT | EGFR | NM_0052 28.3 | + | chr7 | 55272981 | > | 3 | SLC34A2>>EGFR | 6 |
| TSC2<>SLC34A2 | <> | TSC2 | NM_0005 48.3 | + | chr16 | 2110699 | < |  | SLC34A2 | NM_0064 24.2 | + | chr4 | 25678927 | > | 3 | TSC2<>SLC34A2 | 6 |
| ROS1>>AFF3 | >> | ROS1 | NM_0029 44.2 | - | chr6 | 11773074 5 | > | AAAATGCA GACCTTCCA ACTGCTCCC TTTGCTTC | AFF3 | NM_0022 85.2 | - | chr2 | 10070323 7 | > | 2 | ROS1>>AFF3 | 500 |

Example 5: Final fusion gene determination

The fusion gene template was produced based on reads having a supporting pair count of 2 or more, derived from Example 3. Based on this, the supporting read count was determined, and then the fusion gene candidate group having a supporting read count of 5 or more in the first read and/or the second read was finally determined as a fusion gene.
As can be seen from Table 4, the result showed that fusion genes that cannot be detected by the conventional well-known program can be detected.

[Table 4]

Comparison in gene detection result between present invention and conventional program

| Fusion gene (by FISH) | Fusion gene (by program) | Sample | FindingFusion | FACTERA |
|---|---|---|---|---|
| ROS1 | SLC34A:ROS1 | FFPE6 | O | O |
| ROS1 | HSF2:ROS1 / ROS1:VGLL2 | FFPE9 | O | O |
| RET | KIF5B:RET | FFPE22 | O | X |
| RET | CCDC6:RET | FFPE24 | O | X |
| RET | CCDC6:RET | FFPE43 | O | O |
| ALK | EML4:ALK | FFPE17 | O | O |
| ALK | EML4:ALK | FFPE28 | O | O |
| ALK | EML4:ALK | FFPE29 | O | O |
| ALK | EML4:ALK | FFPE37 | O | O |
| ALK | EML4:ALK | FFPE45 | O | O |
| ALK | EML4:ALK | FFPE50 | O | O |
| ALK | EML4:ALK | FFPE52 | O | O |
| ALK | EML4:ALK | FFPE53 | O | O |
| ALK | EML4:ALK | FFPE54 | O | O |
| ALK | HIP:ALK | FFPE56 | O | O |

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

[Industrial Applicability]

The method of detecting a gene rearrangement through NGS according to the present invention is advantageously capable not only of detecting a gene rearrangement through reads obtained using NGS, but also of accurately identifying even the directions of gene rearrangement, and the positions of microhomology sequences, externally inserted sequences and the gene rearrangement in base-pair units, performing detection with high accuracy on concordant read pairs, which cannot be detected by conventional methods, and reducing the time taken for detection owing to the possibility of detection only in certain cancer- or tumor-associated genes. Thus, the method of the present invention is useful for effectively detecting gene rearrangements in cancer samples.

**Claims**

1. A method of detecting a gene rearrangement in a sample comprising:

   (a) acquiring a library including a plurality of nucleic acid molecules from a sample of a subject;
   (b) bringing the library into contact with a plurality of bait sets to enrich the library with respect to a preselected sequence to provide a selected nucleic acid molecule and thereby provide a library catch;
   (c) acquiring reads from the nucleic acid molecules of the library catch through next-generation sequencing;
   (d) arranging the reads by an alignment method; and
   (e) analyzing the arranged reads to detect a gene rearrangement,

   wherein the analysis method comprises extracting the arranged reads to analyze sequence similarity.

2. The method according to claim 1, wherein the step of extracting the reads comprises extracting the reads with information of a region of interest.

3. The method according to claim 1, wherein step (e) comprises extracting the reads and then separating into discordant read pairs and concordant read pairs.

4. The method according to claim 3, wherein the separation of the discordant read pairs and the concordant read pairs is carried out by matching reference gene information to the reads.

5. The method according to claim 3, further comprising, after separation of the discordant read pairs in step (e), finding a matching region of a second read that forms a pair using a soft-clip segment portion of a first read as a query, or finding a matching region of the first read that forms a pair using a soft-clip segment portion of the second read as a query.

6. The method according to claim 3, further comprising, after the separation of the concordant read pairs in step (e), finding a matching region of a second read that forms a pair using a soft-clip segment portion of a first read as a query, or finding a matching region of the first read that forms a pair using a soft-clip segment portion of the second read as a query, while assuming, as a virtual second read, a secondary mapping region obtained by determining whether or not there is mapping to other genes with reference to secondary alignment tag information of the first read.

7. The method according to claim 5, further comprising deriving the read, the matching region of which is found, as a gene rearrangement candidate group to determine a supporting pair count.

8. The method according to claim 6, further comprising deriving the read, the matching region of which is found, as a gene rearrangement candidate group to determine a supporting pair count.

9. The method according to claim 7 or 8, further comprising integrating the supporting pair count.

10. The method according to claim 9, wherein the step of integrating the supporting pair count comprises increasing the supporting pair count when the supporting pair counts are simultaneously determined for the discordant read pair and the concordant read pair.

11. The method according to any one of claims 5 to 10, wherein the supporting pair count is determined to be different when a position of the gene rearrangement is different, although a type of the gene rearrangement is identical.

12. The method according to claim 3, wherein step (e) further comprises arranging the reads not extracted as gene rearrangement candidate reads for further analysis.

13. The method according to claim 12, wherein step (e) further comprises producing a gene rearrangement template based on the gene rearrangement candidate group derived in claim 5 or 6.

14. The method according to claim 1, wherein step (e) further comprises analyzing sequence similarity of reads arranged in claim 12 to determine a supporting read count.

15. The method according to claim 14, wherein the supporting read count is determined as a number of reads that are mapped while passing a breakpoint of the gene rearrangement in the gene rearrangement template after performing blast using the reads arranged in claim 12 as blastdb and using the gene rearrangement template produced in claim 13 as a query.

16. The method according to claim 12, wherein a read unextracted in step (e) is a read present within 500 bp in a 5' direction and a 3' direction from a position of the gene rearrangement candidate group.

17. The method according to claim 16, wherein the read has a soft-clip segment.

18. The method according to claim 1, wherein the step of detecting the gene rearrangement comprises determining, as a gene rearrangement, when the supporting read count is 5 or more.

19. A computer system comprising a computer-readable medium encoded with a plurality of instructions for controlling a computing system to perform a method of detecting a gene rearrangement using next-generation sequencing (NGS),
wherein the method comprises:

   (a) acquiring a library including a plurality of nucleic acid molecules from a sample of a subject;
   (b) bringing the library into contact with a plurality of bait sets to enrich the library with respect to a preselected sequence to provide a selected nucleic acid molecule and thereby provide a library catch;
   (c) acquiring reads from the nucleic acid molecules of the library catch through next-generation sequencing;
   (d) arranging the reads by an alignment method; and
   (e) analyzing the arranged reads to detecting a gene rearrangement, wherein the analysis method includes extracting the arranged reads to analyze sequence similarity.

20. The method according to any one of claims 1 to 18, wherein the sample is selected from the group consisting of: one or more premalignant or malignant cells; cells selected from solid tumors, soft tissue tumors or metastatic lesions; tissue or cells from surgical resections; histologically normal tissue; at least one blood tumor cell (CTC); and blood samples from the same subject having or at risk of developing a normal adjacent tumor (NAT) and a tumor.

FIG. 1

## Specimen

Specimen derived from cancer tissue is a mixture of normal cells and cancer cells, and cells undergoing fusion and cells undergoing no fusion are mixed in cancer cells.

ChrA        ChrB

Random fragmentation

## Library preparation

Random fragmentation is performed after DNA extraction. A plurality of DNAs from cells undergoing fusion are fragmented to various lengths to produce various reads (data) demonstrating fusion as shown in the drawing at right.

## Sequencing

Sequencing data (reads) are produced at both ends of a single DNA fragment, which is called a "paired-end method", and paired reads are derived from the same DNA fragment.

read1

read2

read1                              read2
TTACAGGCCT----------CTTGAGAGCC
read pair derived from non-fusion region(chrB)

ATTCAGGCCT----------CTTGAGAGCC
read pair undergoing fusion, Concodant pair (chrB)

ATTTCCGGAC----------CTTGAGAGCC
read pair undergoing fusion, Concodant pair (chrA-chrB)

ATTTTCGGAC----------AGAGTTCGCC
read pair undergoing fusion, Concodant pair (chrA)

ATTTTCGGAC----------AGAGTTCGCC
read pair derived from non-fusion region(chrA)

These three types of reads provide evidence for fusion between chromosomes A and B.
- The level of evidence varies depending on how many of three types of reads are found.
- In addition, whether or not the number of reads supporting each kind of read is sufficient provides the criteria for the judgment.
- The scoring formula is used to determine presence of the fusion and reliability.

FIG. 2

Translocated chromosome

Read1
Read2

Chr7 | Chr10

Both R1 and R2 are mapped to the same chromosome

Type 1. Concordant read pair having soft clip having no SA information

Both R1 and R2 are mapped to the same chromosome, but R2 has soft clip

Both R1 and R2 are mapped to the same chromosome, but R1 has soft clip

Type 2. Concordant read pair having soft clip having SA information

R1 is mapped to Chr7 and has soft clip

R2 is mapped to Chr10 and has soft clip

Type 3. Discordant read pair having soft clip

Type 4. Discordant read pair having soft clip having no SA information

In case 1, used for support read count operation
In case 2, fusion partner can be detected using SA tag (further described on next chapter)
In case 3, fusion gene and fusion breakpoint can be detected simultaneously
In case 4, fusion gene can be detected but breakpoint cannot be detected, used for support read count operation

FIG. 3

chr7

100 |     150 |     200 |     250 |

Read1

Read2

**Read1>Chr7:100-200 (100M)** : Mapping to 100-200 region of Chr7, 100M means all 100 are matched to reference genome

**Read2>Chr7:150-250 (80M20S)** : Mapping to 150-250 region of Chr7, but means only 80 basepairs are matched and 20 basepairs are soft-clipped

Read2 has additional information of **SA:Chr10:300-400 (80S20M)**. This means secondary alignment (SA) and means that this read2 can be mapped to 300-400 region of Chr10 (with 80 softclips and 20 matches).

chr10

250 |     300 |     350 |     400 |

Virtual Read1

Virtual_Read1>Chr10:300-400 (80S20M): Virtual Read1 (mate-read of Read2) is formed with reference to SA tag of Read2 to form **new virtual discordant read pair** of Virtual-Read1-Read2.

**Chr7:230  Chr10:380**

100 |     150 |     200 |     : 400 |     450 |     500 |

Virtual Read1

Read2

FIG. 4

Fig. 5

FIG: 6

## INTERNATIONAL SEARCH REPORT

| International application No. |
| --- |
| **PCT/KR2018/009086** |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
    because they relate to subject matter not required to be searched by this Authority, namely:

2. ☐ Claims Nos.:
    because they relate to parts of the international application that do not comply with the prescribed requirements to such an extent that no meaningful international search can be carried out, specifically:

3. ☒ Claims Nos.: **11, 15, 20**
    because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.
      ☐ The additional search fees were accompanied by the applicant's protest but the applicable protest fee was not paid within the time limit specified in the invitation.
      ☐ No protest accompanied the payment of additional search fees.

Form PCT/ISA/210 (continuation of first sheet (2)) (January 2015)

## INTERNATIONAL SEARCH REPORT

| International application No. |
| --- |
| **PCT/KR2018/009086** |

**A.    CLASSIFICATION OF SUBJECT MATTER**

*G06F 19/24(2011.01)i, G06F 19/20(2011.01)i*

According to International Patent Classification (IPC) or to both national classification and IPC

**B.    FIELDS SEARCHED**

Minimum documentation searched (classification system followed by classification symbols)
G06F 19/24; C12Q 1/68; C40B 30/04; C40B 30/10; G06F 19/20; G06F 19/22

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: gene rearrangement, library, bait, catch, next generation sequencing, read

**C.    DOCUMENTS CONSIDERED TO BE RELEVANT**

| Category* | Citation of document, with indication, where appropriate, of the relevant passages | Relevant to claim No. |
| --- | --- | --- |
| Y | HAYES, M. et al., "Bellerophon: a Hybrid Method for Detecting Interchromo-somal Rearrangements at Base Pair Resolution Using Next-generation Sequencing Data", BMC Bioinformatics, 2013, vol. 14, pages 1-9<br>See abstract; pages 1-5; figures 1 and 3. | 1-10,12-14,16-19 |
| Y | US 2010-0029498 A1 (GNIRKE, Andreas et al.) 04 February 2010<br>See abstract; claim 1. | 1-10,12-14,16-19 |
| Y | SCHRDER, J. et al., "Socrates: Identification of Genomic Rearrangements in Tumour Genomes by Re-aligning Soft Clipped Reads", Bioinformatics, 2014, vol. 30, no. 8, pages 1064-1072<br>See pages 1066 and 1067. | 12-14,16-18 |
| Y | KR 10-2014-0024270 A (FOUNDATION MEDICINE, INC.) 28 February 2014<br>See claims 1 and 47. | 1-10,12-14,16-19 |
| A | KR 10-2017-0000743 A (SAMSUNG LIFE PUBLIC WELFARE FOUNDATION) 03 January 2017<br>See claims 1-9. | 1-10,12-14,16-19 |
| PX | KR 10-1867011 B1 (NGENEBIO) 14 June 2018<br>See claims 1, 2, 4, 10-14 and 16-19. | 1-10,12-14,16-19 |

☐   Further documents are listed in the continuation of Box C.          ☒   See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 NOVEMBER 2018 (15.11.2018) | **15 NOVEMBER 2018 (15.11.2018)** |

| Name and mailing address of the ISA/KR | Authorized officer |
| --- | --- |
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2018/009086**

| Patent document cited in search report | Publication date | Patent family member | Publication date |
|---|---|---|---|
| US 2010-0029498 A1 | 04/02/2010 | EP 2245198 A1 | 03/11/2010 |
| | | US 2015-0126377 A1 | 07/05/2015 |
| | | WO 2009-099602 A1 | 13/08/2009 |
| KR 10-2014-0024270 A | 28/02/2014 | AU 2011-352070 A1 | 18/07/2013 |
| | | AU 2017-203322 A1 | 08/06/2017 |
| | | AU 2017-208342 A1 | 17/08/2017 |
| | | AU 2017-239591 A1 | 02/11/2017 |
| | | AU 2017-276313 A1 | 18/01/2018 |
| | | AU 2018-201701 A1 | 05/04/2018 |
| | | AU 2018-203684 A1 | 14/06/2018 |
| | | AU 2018-211304 A1 | 23/08/2018 |
| | | BR 112013016708 A2 | 04/10/2016 |
| | | CA 2823621 A1 | 05/07/2012 |
| | | EP 2659003 A1 | 06/11/2013 |
| | | EP 3225697 A2 | 04/10/2017 |
| | | EP 3225697 A3 | 22/11/2017 |
| | | IL 227195 A | 29/08/2013 |
| | | JP 2014-507133 A | 27/03/2014 |
| | | JP 2017-077244 A | 27/04/2017 |
| | | JP 2018-134083 A | 30/08/2018 |
| | | JP 6054303 B2 | 27/12/2016 |
| | | SG 191818 A1 | 30/08/2013 |
| | | US 2012-0208706 A1 | 16/08/2012 |
| | | US 9340830 B2 | 17/05/2016 |
| | | WO 2012-092426 A1 | 05/07/2012 |
| KR 10-2017-0000743 A | 03/01/2017 | CN 107408162 A | 28/11/2017 |
| | | KR 10-1881838 B1 | 25/07/2018 |
| | | SG 11201707653 A | 30/10/2017 |
| | | WO 2016-208826 A1 | 29/12/2016 |
| KR 10-1867011 B1 | 14/06/2018 | NONE | |

Form PCT/ISA/210 (patent family annex) (January 2015)

**EP 3 667 672 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RABBITTS, T.H. et al.** *Nature,* vol. 372, 143-1491994
- **DAVID, R. et al.** *Cell Physiol. Biochem.,* 2015, vol. 37 (1), 77-93
- **TAKASHI, K. et al.** *Ann Oncol.,* 2014, vol. 25 (1), 138-42
- **FEUK, L. et al.** *Nat Rev Genet.,* 2006, vol. 7 (2), 85-97
- **CHIANG, D.Y. et al.** *Nat Methods.,* 2009, vol. 6 (1), 99-103
- **CHEN, K. et al.** *Nat Methods.,* 2009, vol. 6 (9), 677-81
- **METZKER, M.** *Nature Biotechnology Reviews,* 2010, vol. 11, 31-46
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98